# EUROPEAN PATENT SPECIFICATION

(11) **EP 1 003 875 B1**
(45) Date of publication and mention of the grant of the patent: **27.12.2006**
(21) Application number: 98946991.1
(22) Date of filing: 14.09.1998
(51) Int. Cl.: C12N 15/31, C12N 15/62, C07K 14/315, A61K 39/09, A61K 39/39

(54) **GROUP A STREPTOCOCCAL VACCINES**
GRUPPE A STREPTOKOKKEN-IMPFSTOFF
VACCIN A BASE DE STREPTOCOQUES DU GROUPE A

(30) Priority: 12.09.1997 US 58635 P
(43) Date of publication of application: 31.05.2000
(73) Proprietor: UNIVERSITY OF TENNESSEE RESEARCH FOUNDATION, Knoxville, TN 37996-1527 (US)
(72) Inventor: DALE, James, B., Memphis, TN 38111 (US)
(74) Representative: Gowshall, Jonathan Vallance
(86) International application number: PCT/US1998/019100
(87) International publication number: WO 1999/013084

(56) References cited:
- WO-A-94/06421
- BEACHEY E H ET AL: "PROTECTIVE IMMUNOGENICITY AND T LYMPHOCYTE SPECIFICITY OF A TRIVALENT HYBIRD PEPTIDE CONTAINING NH2-TERMINAL SEQUENCES OF TYPES 5, 6, AND 24 M PROTEINS SYNTHESIZED IN TANDEM" JOURNAL OF EXPERIMENTAL MEDICINE, vol. 166, no. 3, 1 September 1987, pages 647-656, XP000618953
- DALE J B ET AL: "Recombinant, octavalent group A streptococcal M protein vaccine" VACCINE, vol. 14, no. 10, July 1996, page 944-948 XP004057623 cited in the application
- DALE J B ET AL: "RECOMBINANT TETRAVALENT GROUP A STREPTOCOCCAL M PROTEIN VACCINE" JOURNAL OF IMMUNOLOGY, vol. 151, no. 4, 15 August 1993, pages 2188-2194, XP000645174 cited in the application
- BEACHEY E H ET AL: "OPSONIC ANTIBODIES EVOKED BY HYBRID PEPTIDE COPES OF TYPES 5 AND 24 STREPTOCOCCAL M PROTEINS SYNTHESIZED IN TANDEM" JOURNAL OF EXPERIMENTAL MEDICINE, vol. 163, no. 6, June 1986, pages 1451-1458, XP000645526
- WISTEDT A C ET AL: "Identification of a plasminogen-binding motif in PAM, a bacterial surface protein" MOLECULAR MICROBIOLOGY, vol. 18, 1995, pages 569-578, XP002092777 GB

## Description

### CROSS-REFERENCE TO RELATED APPLICATIONS

This application claims the benefit of United States Provisional Application No. 60/058,635, filed September 12, 1997, which application is incorporated by reference in its entirety.

### TECHNICAL FIELD

The present invention provides pharmaceutical compositions and methods, and in particular, vaccines for use in preventing Group A streptococcal infections.

### BACKGROUND OF THE INVENTION

Streptococci are a group of bacteria with the capacity to grow in chains. Many varieties are part of the normal bacterial flora in humans and are not especially harmful. However, a particular group of streptococcal bacteria, called group A and represented by *Streptococcus pyogenes,* is a human pathogen. Briefly, group A streptococci cause a variety of human illnesses, ranging from uncomplicated pharyngitis and pyoderma to life-threatening infections associated with toxic shock syndrome, deep tissue invasion and sepsis. In some individuals, untreated streptococcal pharyngitis may be followed by acute rheumatic fever. In recent years there has been a dramatic increase in the incidence of severe streptococcal infections (Davies et al., "Invasive group A streptococcal infections in Ontario, Canada. Ontario group A Streptococcal study group," *N. Engl. J. Med. 335*:547-554, 1996) and in the incidence of rheumatic fever (Veasey et al., "Resurgence of acute rheumatic fever in the intermountain region of the United States." *N. Eng. J Med. 316*:421-427, 1987).

Although streptococcal infections can be generally treated with antibiotics, in at least 4% of cases the infection leads to acute rheumatic fever. This disease is particularly prevalent in developing countries such as India, where millions of school-age children are affected.

The present invention provides new Group A streptococcal vaccines with enhanced immunogenicity, and further, provides other related advantages.

### SUMMARY OF THE INVENTION

Briefly stated, the present invention provides immunogenic synthetic fusion polypeptides which stimulate an immune response against Group A streptococci. Within one aspect such polypeptides comprise (a) at least two immunogenic polypetides from a Group A streptococci of at least 10 amino acids in length which are capable of stimulating an immune response against Group A streptococci, and a peptide C terminal to the immunogenic polypeptide which protects the immunogenicity of the immunogenic portion. Within preferred embodiments, the C-terminal peptide is not required to stimulate an immune response against Group A streptococci and hence, may be an inconsequential non-immunogenic peptide, or a reiterated immunogenic polypeptide. Within certain embodiments, the immunogenic polypeptide can be obtained from a wide variety of Group A streptococci (ranging from "1" to greater than "90"), including for example, Types 1, 1.1, 2, 3, 4, 5, 6, 11, 12, 13, 14, 18, 19, 22, 24, 28, 30, 48, 49, 52, 55 and 56.

Within other aspects of the present invention, vaccinating agents are provided for promoting an immune response against Group A streptococci, comprising (a) at least two immunogenic polypetides from a Group A streptococci of at least 10 amino acids in length which are capable of stimulating a protective immune response against Group A streptococci, and (b) a peptide C terminal to the immunogenic polypeptide which protects the immunogenicity of the immunogenic portion, wherein the C-terminal peptide is not required to stimulate an immune response against Group A streptococci. As above, the polypeptide may selected from a wide variety of Group A streptococci (ranging from "1" to greater than "90"), including for example, types 1.1, 2, 3, 4, 5, 6, 11, 12, 13, 14, 18, 19, 22, 24, 28, 30, 48. 49. 52, 55 and 56. Within certain further embodiments, the vaccinating agent may further comprise an adjuvant, such as, for example, alum, Freund's adjuvant, and/or an immunomodulatory cofactor (*e.g*., IL-4, IL-10, γ-IFN, or IL-2, IL-12 or IL-15).

Also provided are methods for vaccinating a host against Group A streptococci infections, comprising administering a vaccinating agent as described above.

These and other aspects of the present invention will become evident upon reference to the following detailed description and attached drawings. In addition, various references are set forth herein which describe in more detail certain procedures or compositions (e.g., plasmids, etc.), and are therefore incorporated by reference in their entirety.

### BRIEF DESCRIPTION OF THE DRAWINGS

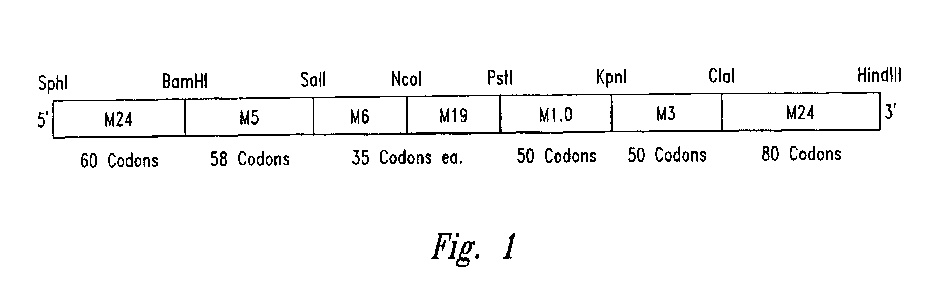

Figure 1 is a schematic of the hexavalent vaccine indicating the length of each emm gene fragment and the restriction sites that were synthesized into the original PCR primers. Each of the emm gene fragments starts at the first codon that encodes the mature native protein except the emm3 fragment, which represents codons 21-70.

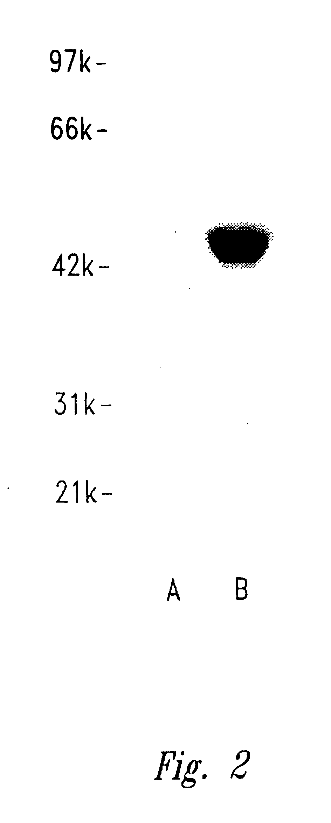

Figure 2 is a SDS-polyacrylamide gel electrophoresis of the purified hexavalent protein stained with coomassie blue. Computer-assisted image analysis of the stained protein bands indicated that the hexavalent protein (M.W. 45 kDa) accounted for 90.3% of the total protein in the sample.

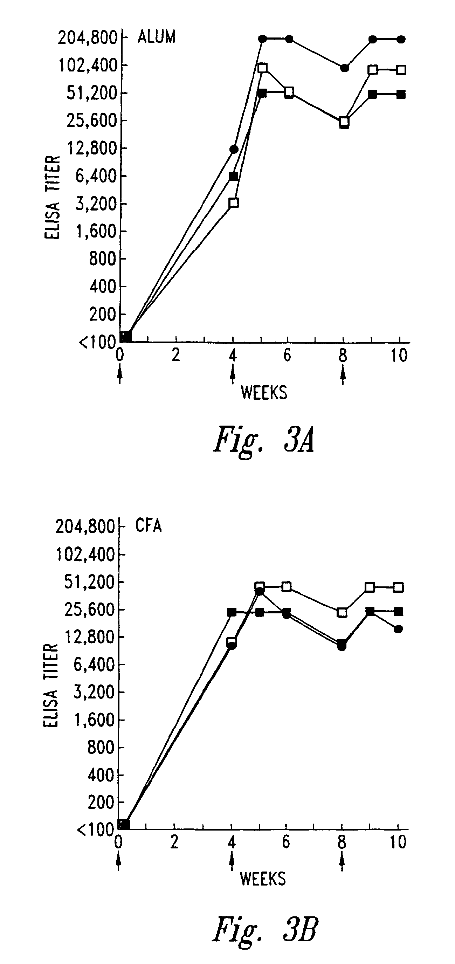

Figures 3A and 3B are ELISA's of antisera from three rabbits immunized with the hexavalent vaccine in either alum or CFA. Titers are expressed as the inverse of the last dilution of serum that resulted in an O.D. of >0.1. The ELISA antigen was the purified hexavalent protein. Each symbol represents serum from one rabbit.

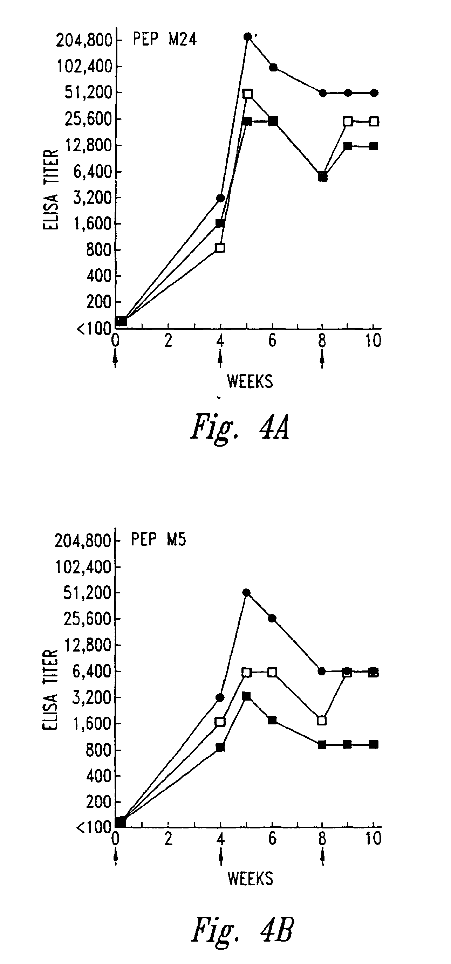

Figures 4A-4F are ELISA's of antisera from rabbits immunized with the hexavalent protein in alum. Titers were determined using the purified pepsin-extracted M proteins (pep M) from the respective serotypes of group A streptococci. Each symbol represents one of three immunized rabbits.

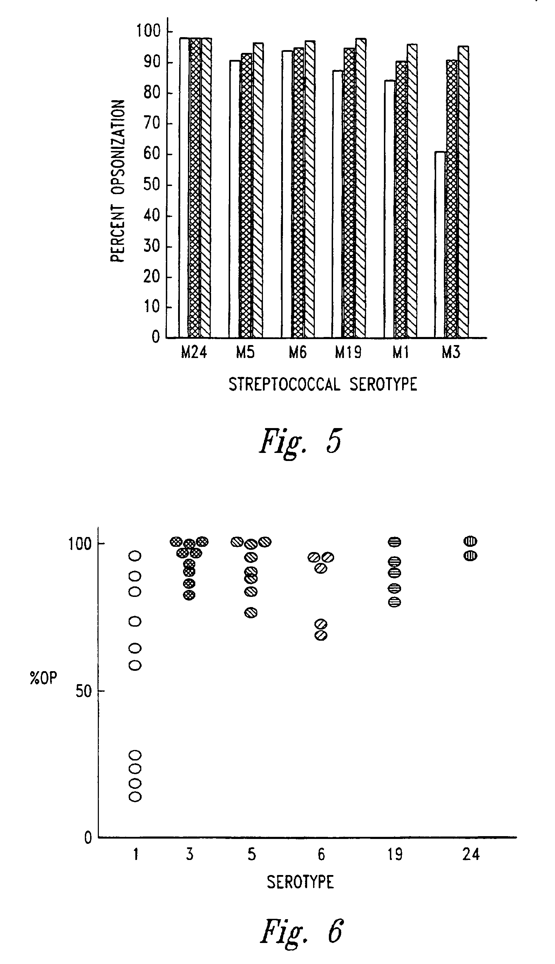

Figure 5 depicts *in vitro* opsonization assays of antisera from rabbits immunized with the hexavalent protein in alum. Rotation mixtures consisted of the test organism, 0.1 ml of immune serum, and 0.4 ml of nonimmune human blood. The mixture was rotated for 45 minutes and the percentage of PMNs that had ingested or were associated with streptococci was estimated by microscopic counts of stained smears. In each assay, the preimmune serum resulted in <10% percent opsonization. Each different bar represents serum from one of the three immunized rabbits.
Figure 6 is a graph which depicts opsonization of different strains within the same serotype of group A streptococci promoted by hexavalent rabbit antisera. Each symbol represents a strain of group A streptococci of the serotype indicated on the horizontal axis. Opsonization assays were performed as described below in the Examples.

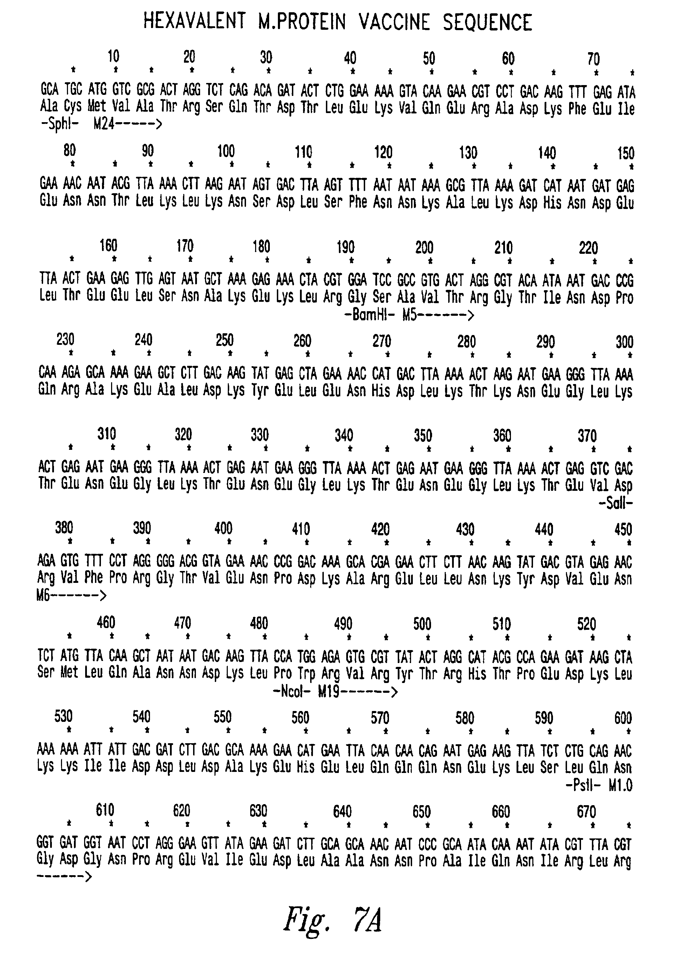

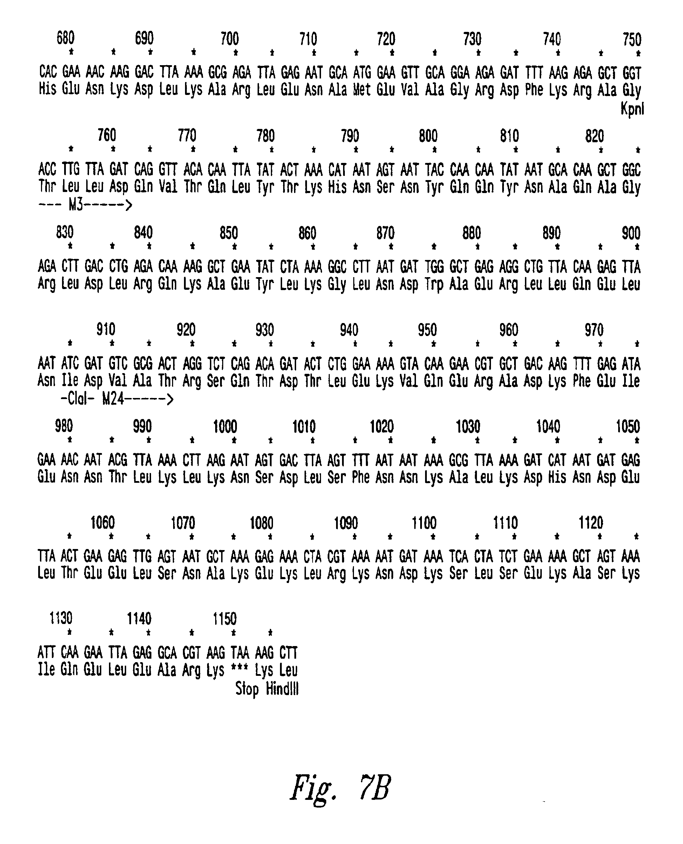

Figure 7 is a sequence of the hexavalent M protein vaccine (SEQ ID NO:15 and SEQ ID NO:16).

### DETAILED DESCRIPTION OF THE INVENTION

### DEFINITIONS

Prior to setting forth the invention, it may be helpful to an understanding thereof to first set forth definitions of certain terms that will be used hereinafter.

"Vaccinating Agent" refers to a composition which is capable of stimulating a protective immune response within the host which receives the vaccinating agent. The vaccinating agent may be either protein, or, DNA-based (e.g., a gene delivery vehicle). Within further aspects, a prokaryotic host may be generated to be a vaccinating agent, and designed to express an immunogenic polypeptide or multivalent construct of the present invention *(see, e.g.,* U.S. Application No. 07/540,586).

"Gene delivery vehicle" refers to a recombinant vehicle, such as a recombinant viral vector, a nucleic acid vector (such as plasmid), a naked nucleic acid molecule such as genes, a nucleic acid molecule complexed to a polycationic molecule capable of neutralizing the negative charge on the nucleic acid molecule and condensing the nucleic acid molecule into a compact molecule, a nucleic acid associated with a liposome (Wang et al., *PNAS* 84:7851, 1987), a bacterium, and certain eukaryotic cells such as a producer cell, that are capable of delivering a nucleic acid molecule having one or more desirable properties to host cells in an organism.

As noted above, the present invention provides vaccinating agents suitable for preventing Group A streptococcal infections. Briefly, as described in more detail below it has been discovered that, in order to optimize the immunogenicity of all aspects of a multivalent vaccine. Within one aspect of the invention, immunogenic synthetic fusion polypeptides which stimulate an immune response against Group A streptococci are provided. Such polypeptides generally comprise (a) at least two immunogenic polypetides from a Group A streptococci of at least 10 amino acids in length which are capable of stimulating an immune response against Group A streptococci, and (b) a peptide C terminal to the immunogenic polypeptide which protects the immunogenicity of the immunogenic portion, wherein the C-terminal peptide is not required to stimulate an immune response against Group A streptococci. Particularly preferred protective peptides are generally at least ten amino acids in length, and may be 30 amino acids or longer.

### Identification of Immunogenic Polypeptides, for Use in Vaccinating Agents

Immunogenic polypeptides suitable for use within the present invention may be readily identified and generated given the disclosure of the subject application *(see also* Dale and Beachey, *J. Exp. Med. 163*:1191-1202; 1986: Beachey et al., *Nature 292*:457-459, 1981; Dale et al., *J. Immunol. 151*:2188-2194; 1993; and U.S. Patent Nos. 4,454,121; 4,521,334; 4,597,967; 4,705,684; 4,919,930; and 5,124,153). Particularly preferred polypeptides can be obtained within the 50 amino acid residues of the N-terminus of an M protein.

Serotypes of Group A streptococci can be readily obtained from clinical isolates, from university collections (e.g., Rockefeller University Collection, 1230 York Avenue, New York, NY) or from depositories such as the American Type Culture Collection (10801 University Boulevard, Manassas, Virginia). Furthermore, sequences for Group A streptococci serotypes are available from the Centers for Disease Control. Atlanta, Georgia.

### A. Identification of Opsonic Epitopes of M Proteins

To demonstrate directly that opsonic M protein epitopes could be separated from autoimmune epitopes, peptides are copied from various serotypes (*e.g*., the amino-terminal 20-50 amino acids of M5 (Beachey et al., "Purification and properties of M protein extracted from group A streptococci with pepsin: Covalent structure of the amino terminal region of the type 24 M antigen," *J. Exp. Med. 145*:1469-1483, 1977). SM5(1-20) failed to react with affinity purified pep M5 heart-reactive antibodies (Beachey et al., "Purification and properties of M protein extracted from group A streptococci with pepsin: Covalent structure of the amino terminal region of the type 24 M antigen," *J. Exp. Med. 145*:1469-1483, 1977). Rabbits immunized with SM5(1-20) coupled to tetanus toxoid developed high titers of antibodies against pep M5 that opsonized type 5 streptococci (Beachey et al., "Purification and properties of M protein extracted from group A streptococci with pepsin: Covalent structure of the amino terminal region of the type 24 M antigen," *J. Exp. Med. 145*:1469-1483, 1977). Most importantly, none of the immune sera crossreacted with human myocardium.

### B. Tissue-Crossreactive Epitopes of M Proteins

M proteins evoke antibodies that crossreact with a variety of human tissues and antigens within those tissues (Baird et al., "Epitopes of group A streptococcal M protein shared with antigens of articular cartilage and synovium," *J. Immunol. 146*:3132-3137, 1991; Bronze, M.S. and Dale, J.B., "Epitopes of streptococcal M proteins that evoke antibodies that cross-react with human brain," *J. Immunol. 151*:2820-2828., 1993; Dale, J.B. and Beachey E.H., "Protective antigenic determinant of streptococcal M protein shared with sarcolemmal membrane protein of human heart," *J. Exp. Med. 156*:1165-1176, 1982). In order to determine cross-reactivity, a series of overlapping peptides is synthesized that copies a selected fragment (e.g., M5), and used to either inhibit or evoke tissue-crossreactive antibodies. For example, the myosin-crossreactive antibodies evoked by pep M5 in rabbits were almost totally inhibited by peptide 84-116 of pep M5. This peptide spans the region between the A and B repeats of M5 and includes the degenerate A6 repeat. Murine and human myosin-crossreactive antibodies reacted with an epitope in peptide 183-189, which is located in the region between the B and C repeats of the intact M5 molecule.

Additional sarcolemmal membrane crossreactive epitopes are localized to peptide 164-197. Several epitopes of M5 that evoked antibodies that crossreacted with articular cartilage and synovium can also be found within the B repeats and the region spanning the A and B repeats of M5. The brain-crossreactive epitopes of M6 that were shared with other M proteins are localized to the B repeat region of the molecule.

Many of the tissue-crossreactive epitopes are shared among types 5, 6, 18 and 19 M proteins (Bronze, M.S. and Dale, J.B., "Epitopes of streptococcal M proteins that evoke antibodies that cross-react with human brain," *J. Immunol. 151*:2820-2828., 1993). Primary structural data reveals that all of these M proteins contain similar sequences within their B repeats (Dale et al., "Recombinant tetravalent group A streptococcal M protein vaccine," *J. Immunol. 151*:2188-2194, 1993; Dale et al., "Recombinant, octavalent group A streptococcal M protein vaccine," *Vaccine 14*:944-948, 1996; Dale et al., "Type-specific immunogenicity of a chemically synthesized peptide fragment of type 5 streptococcal M protein," *J. Exp. Med. 158*:1727-1732, 1983), which is most likely the location of the shared heart- , brain-, and joint-crossreactive epitopes.

It should be emphasized that it is not necessary to localize the tissue-specific epitope, but rather, to first localize protective epitopes and ensure that they are not tissue-reactive.

Once a suitable immunogenic polypeptide for a selected serotype has been identified, it may be, optionally, combined with immunogenic polypeptides from other serotypes, in order to construct a multivalent vaccine. In this regard, preferred vaccines include vaccines developed from a combination of serotypes such as 1, 1.1, 2, 3, 4, 5, 6, 11, 12, 13, 14, 18, 19, 22, 24, 28, 30, 48, 49, 52 and 56 (for serotype 30 see Nakashima et al., *Clinic Infec. Dis.* 25:260, 1997). Representative examples include vaccine such as 24, 5, 6, 19, 1, 3, X; and 1, 3, 5, 6, 18, 19, 22, 24, 28. 30, and X, wherein X is the C-terminal protective polypeptide.

### PREPARATION OF VACCINATING AGENTS

Vaccinating agents of the present invention can be synthesized chemically (see, *e.g.,* Beachey et al., *Nature* 292:457-459, 1981), or generated recombinantly. For recombinant production, PCR primers can be synthesized to amplify desired 5'sequences of each emm gene, and each primer is extended to contain a unique restriction enzyme site used to ligate the individual PCR products in tandem.

As noted above, the C-terminal portion of the vaccinating agent is constructed so as to contain a selective portion that can be lost or cleaved *in vivo* without affecting the efficacy of the vaccine. This may be accomplished by, for example, including an inconsequential non-immunogenic polypeptide at the end, or, including an immunogenic polypeptide that does not adversely impact the efficiency of the vaccine (*e.g*., a reiterated immunogenic polypeptide may be included at the end of the vaccine). Furthermore, protective antigens from unrelated pathogens can also be combined into a single polypeptide, which may circumvent the need for carriers. Vaccines against some pathogens might include T and B cell epitopes originally derived from different proteins on the same hybrid construct. Additionally, multivalent hybrid proteins may be sufficient conjugates in carbohydrate vaccines, such as those for *S. pneumoniae, H. influenza* B or group B streptococci.

For protein expression, the multivalent genes are ligated into any suitable replicating plasmid which is used to transform an appropriate prokaryote host strain. Prokaryotes include gram negative or gram positive organisms, for example *E. coli* or bacilli. Suitable prokaryotic hosts cells for transformation include, for example, *E. coli, Bacillus subtilis, Salmonella typhimurium,* and various other species within the genera *Pseudomonas, Streptomyces,* and *Staphylococcus.*

Expression vectors transfected into prokaryotic host cells generally comprise one or more phenotypic selectable markers such as, for example, a gene encoding proteins that confer antibiotic resistance or that supplies an autotrophic requirement, and an origin of replication recognized by the host to ensure amplification within the host. Other useful expression vectors for prokaryotic host cells include a selectable marker of bacterial origin derived from commercially available plasmids. This selectable marker can comprise genetic elements of the cloning vector pBR322 (ATCC 37017). Briefly, pBR322 contains genes for ampicillin and tetracycline resistance and thus provides simple means for identifying transformed cells. The pBR322 "backbone" sections are combined with an appropriate promoter and a mammalian ETF structural gene sequence. Other commercially available vectors include, for example, pKK223-3 (Pharmacia Fine Chemicals, Uppsala, Sweden), pQE30 and pGEM1 (Promega Biotec, Madison, WI, USA).

Common promoter sequences for use within prokaryotic expression vectors include β-lactamase (penicillinase), lactose promoter system (Chang et al., *Nature 275*:615, 1978; and Goeddel et al., *Nature 281*:544, 1979), tryptophan (trp) promoter system (Goeddel et al., *Nucl. Acids Res. 8*:4057, 1980; and EPA 36,776) and tac promoter (Sambrook et al., Molecular Cloning: A Laboratory Manual, Cold Spring Harbor Laboratory, (1989)). A particularly useful prokaryotic host cell expression system employs a phage λ P_{L} promoter and a cI857ts thermolabile repressor sequence. Plasmid vectors available from the American Type Culture Collection that incorporate derivatives of the λ P_{L} promoter include plasmid pHUB2 (resident in *E. coli* strain JMB9 (ATCC 37092)) and pPLc28 (resident in *E. coli* RR1 (ATCC 53082)).

Transformation of the host strains of *E*. *coli* is accomplished by electroporation using standard methods (Dale et al., "Recombinant tetravalent group A streptococcal M protein vaccine," *J. Immunol. 151*:2188-2194, 1993; Dale et al., "Recombinant, octavalent group A streptococcal M protein vaccine," *Vaccine 14*:944-948, 1996). Successful transformants are identified by colony blots using rabbit antisera raised against one of the native M proteins or a synthetic peptide copy of the amino-terminus of one of the M proteins included in the multivalent protein.

The molecular size and antigenicity of the recombinant protein expressed by selected clones are determined by performing Western blots of extracts of *E. coli* (Dale et al., "Recombinant tetravalent group A streptococcal M protein vaccine," *J. Immunol. 151*:2188-2194, 1993) using rabbit antisera raised against each native M protein purified from pepsin extracts of live streptococci (Beachey et al., "Purification and properties of M protein extracted from group A streptococci with pepsin: Covalent structure of the amino terminal region of the type 24 M antigen," *J. Exp. Med. 145*:1469-1483, 1977). The multivalent gene is sequenced by the dideoxy-nucleotide chain termination method to confirm that each gene fragment is an exact copy of the native emm sequence.

### GENE-DELIVERY VEHICLE-BASED VACCINES

Injection of mammals with gene delivery vehicles (*e.g.,* naked DNA) encoding antigens of various pathogens has been shown to result in protective immune responses (Ulmer et al., *Science 259*:1745-9, 1993; Bourne et al., *J Infect. Dis. 173*:800-7, 1996; Hoffman et al., *Vaccine 12*:1529-33, 1994). Since the original description of *in vivo* expression of foreign proteins from naked DNA injected into muscle tissue (Wolff et al., *Science 247*:1465-8, 1990), there have been several advances in the design and delivery of DNA for purposes of vaccination.

The M protein vaccines described above are ideally suited for delivery via naked DNA because protective immunity is ultimately determined by antibodies. For example, within one embodiment the multivalent genes are ligated into plasmids that are specifically engineered for mammalian cell expression *(see, e.g.,* Hartikka et al., *Hum Gene Ther 7*:1205-17, 1996, which contains the promoter/enhancer element from cytomegalovirus early gene, the signal peptide from human tissue plasminogen activator and a terminator element from the bovine growth hormone gene). The M protein hybrid genes can be cloned into the plasmid which is used to transfect human cell lines to assure recombinant protein expression. The plasmid is propagated in E. coli and purified in quantities sufficient for immunization studies by cesium chloride gradient centrifugation. Mice are immunized with 50 ug of plasmid in 50 ul saline given intramuscularly into the rectus femoris. Booster injections of the same dose are given at three and six weeks after the initial injection.

A wide variety of other gene delivery vehicles can likewise be utilized within the context of the present invention, including for example, viruses, retrotransposons and cosmids. Representative examples include adenoviral vectors (*e.g.,* WO 94/26914, WO 93/9191; Yei et al., *Gene Therapy 1*:192-200, 1994; Kolls et al., *PNAS 91*(1):215-219, 1994; Kass-Eisler et al., *PNAS 90*(24):11498-502, 1993; Guzman et al., *Circulation 88*(6):2838-48, 1993; Guzman et al., *Cir. Res. 73*(6):1202-1207, 1993; Zabner et al., *Cell 75*(2):207-216, 1993; Li et al., *Hum Gene Ther. 4*(4):403-409, 1993; Caillaud et al., *Eur. J. Neurosci. 5*(10):1287-1291, 1993), adeno-associated type 1 ("AAV-1") or adeno-associated type 2 ("AAV-2") vectors (*see* WO 95/13365; Flotte et al., *PNAS 90*(22):10613-10617, 1993), hepatitis delta vectors, live, attenuated delta viruses and herpes viral vectors *(e.g.,* U.S. Patent No. 5,288.641), as well as vectors which are disclosed within U.S. Patent No. 5,166,320. Other representative vectors include retroviral vectors (*e.g*., EP 0 415 731; WO 90/07936; WO 91/02805; WO 94/03622; WO 93/25698; WO 93/25234; U.S. Patent No. 5,219,740; WO 93/11230; WO 93/10218). Methods of using such vectors in gene therapy are well known in the art, see, for example, Larrick, J.W. and Burck, K.L., *Gene Therapy: Application of Molecular Biology,* Elsevier Science Publishing Co.. Inc., New York, New York, 1991; and Kreigler, M., *Gene Transfer and Expression: A Laboratory Manual,* W.H. Freeman and Company, New York, 1990.

Gene-delivery vehicles may be introduced into a host cell utilizing a vehicle, or by various physical methods. Representative examples of such methods include transformation using calcium phosphate precipitation (Dubensky et al., *PNAS 81*:7529-7533, 1984), direct microinjection of such nucleic acid molecules into intact target cells (Acsadi et al., *Nature 352*:815-818, 1991), and electroporation whereby cells suspended in a conducting solution are subjected to an intense electric field in order to transiently polarize the membrane, allowing entry of the nucleic acid molecules. Other procedures include the use of nucleic acid molecules linked to an inactive adenovirus (Cotton et al., *PNAS 89*:6094, 1990), lipofection (Felgner et al., *Proc. Natl. Acad. Sci. USA 84*:7413-7417, 1989), microprojectile bombardment (Williams et al., *PNAS 88*:2726-2730, 1991), polycation compounds such as polylysine, receptor specific ligands, liposomes entrapping the nucleic acid molecules, spheroplast fusion whereby *E. coli* containing the nucleic acid molecules are stripped of their outer cell walls and fused to animal cells using polyethylene glycol, viral transduction, (Cline et al., *Pharmac. Ther. 29*:69, 1985; and Friedmann et al.. *Science 244*:1275, 1989), and DNA ligand (Wu et al, *J. of Biol. Chem. 264*:16985-16987, 1989), as well as psoralen inactivated viruses such as Sendai or Adenovirus.

Serum from mice immunized with gene delivery vehicles containing multivalent M protein genes are assayed for total antibody titer by ELISA using native M proteins as the antigen. Serum opsonic antibodies are assayed as described above. Protective efficacy of DNA M protein vaccines is determined by direct mouse protection tests using the serotypes of group A streptococci represented in the vaccine.

### FORMULATION AND ADMINISTRATION

For therapeutic use, vaccinating agents can be administered to a patient by a variety of routes, including for example, by intramuscular, subcutaneous, and mucosal routes. The vaccinating agent may be administered as a single dosage, or in multiple units over an extended period of time. Within preferred embodiments, the vaccinating agent is administered to a human at a concentration of 50-300 ug per single site intramuscular injection. Several injections can be given (e.g., three or four) at least one month apart in order to further increase vaccine efficacy.

Typically, the vaccinating agent will be administered in the form of a pharmaceutical composition comprising purified polypeptide in conjunction with physiologically acceptable carriers, excipients or diluents. Such carriers will be nontoxic to patients at the dosages and concentrations employed. Ordinarily, the preparation of such compositions entails combining the vaccinating agent with buffers, antioxidants such as ascorbic acid, low molecular weight (less than about 10 residues) polypeptides, proteins, amino acids, carbohydrates including glucose, sucrose or dextrans, chelating agents such as EDTA, glutathione and other stabilizers and excipients. Neutral buffered saline or saline mixed with conspecific serum albumin are exemplary appropriate diluents.

Within preferred embodiments of the invention, the vaccinating agent is combined with an adjuvant, such as, for example, Freund's adjuvant, alum and the like.

The following examples are offered by way of illustration, and not by way of limitation.

### EXAMPLES

### EXAMPLE 1

### CONSTRUCTION AND EXPRESSION OF A HEXAVALENT FUSION GENE

A hexavalent emm gene was constructed using PCR to amplify specific 5' regions of the six different emm genes (24, 5, 6, 19, 1 and 3) essentially as described previously (Dale et al., "Recombinant tetravalent group A streptococcal M protein vaccine," *J. Immunol. 151*:2188-2194, 1993; Dale et al., "Recombinant, octavalent group A streptococcal M protein vaccine," *Vaccine 14*:944-948, 1996).

Briefly, the multivalent genes are constructed using PCR and primers that specify specific 5' emm gene fragments. The gene fragments may range in size from 30 bp to 300 bp. Chromosomal DNA from each serotype of group A streptococcus is used as the template for the PCR reactions. For the hexavalent emm gene described in the example, the PCR primers are as follows:
M24-1 TS SphI
   5' GGG GGG GCA TCG GTC GCG ACT AGG TCT CAG ACA GAT 3'
   (SEQ ID NO:1)
M24-1 BS BamH1
   5' GGG GGG GGA TCC ACG TAG TTT CTC TTT AGC 3'
   (SEQ ID NO:2)
M5 TS BamH1
   5' GGG GGG GGA TCC GCC GTG ACT AGG GGT ACA 3'
   (SEQ ID NO:3)
M5 BS SalI
   5' GGG GGG GTC GAC CTC AGT TTT TAA CCC TTC 3'
   (SEQ ID NO:4)
M6 TS SalI
   5' GGG GGG GTC GAC AGA GTG TTT CCT AGG GGG 3'
   (SEQ ID NO:5)
M6 BS NcoI
   5' GGG GGG CCA TGG TAA CTT GTC ATT ATT AGC 3'
   (SEQ ID NO:6)
M19 TS NcoI
   5' GGG GGG CCA TGG AGA GTG CGT TAT ACT AGG 3'
   (SEQ ID NO:7)
M19 BS PstI
   5' GGG GGG CTG CAG AGA TAA CTT CTC ATT CTG 3'
   (SEQ ID NO:8)
M1 TS PstI
   5' GGG GGG CTG CAG AAC GGT GAT GGT AAT CCT 3'
   (SEQ ID NO:9)
M1 BS KpnI
   5' GGG GGG GGT ACC AGC TCT CTT AAA ATC TCT 3'
   (SEQ ID NO:10)
M3 TS KpnI
   5' GGG GGG GGT ACC TTG TTA GAT CAG GTT ACA 3'
   (SEQ ID NO:11)
M3 BS ClaI
   5' GGG GGG ATC GAT ATT TAA CTC TTG TAA CAG 3'
   (SEQ ID NO:12)
M24-2 TS ClaI
   5' GGG GGG ATC GAT GTC GCG ACT AGG TCT CAG 3'
   (SEQ ID NO:13)
M24-2 BS HindIII
   5' GGG GGG AAG CTT TTA CTT ACG TGC CTC TAA TTC 3'
   (SEQ ID NO:14)

PCR is performed on the chromosomal template as previously described (Dale et al., "Recombinant tetravalent group A streptococcal M protein vaccine," *J. Immunol. 151*:2188-2194, 1993). To assure ligation of the fragments in the correct orientation and reading frame, each PCR product is purified, ligated, and then subjected to PCR again using the forward primer from the 5' fragment and the reverse primer from the 3' fragment. For example, to construct a hexavalent emm gene containing DNA sequences from types 24, 5, 6, 19, 1, and 3 M proteins, the M24 and M5 gene fragments are amplified by PCR using the primers described above. The PCR products are purified from agarose gels, cut with the appropriate restriction enzyme, and ligated together (Dale et al., "Recombinant tetravalent group A streptococcal M protein vaccine." *J. Immunol. 151*:2188-2194. 1993; Dale et al., "Recombinant, octavalent group A streptococcal M protein vaccine," *Vaccine 14*:944-948, 1996). The ligation mixture is then amplified by PCR using the forward M24 primer and the reverse M5 primer. The resulting product of the appropriate size is then purified and ligated to the M6 and M19 gene fragment that was similarly constructed. After the final ligation reaction, the entire gene is amplified again by PCR, cut with the appropriate restriction enzymes and ligated into a suitable expression vector. For the addition of the reiterated M24 gene fragment in the 3' location, the plasmid was purified from the host E. coli and a new PCR product from emm 24 was force cloned into the 3' PstI restriction site.

The hexavalent gene was sequenced by the dideoxy-nucleotide chain termination method to confirm that each gene fragment was an exact copy of the respective native emm sequence.

### EXAMPLE 2

### PURIFICATION OF A HEXAVALENT VACCINE

### A. Purification

Transformed *E. coli* were grown in a shaking incubator to log phase in 11 of LB containing 100 ug/ml ampicillin and 25 ug/ml kanamycin. IPTG (2mM) was added for the final four hours of growth. The cell pellet was suspended in 30 ml PBS and lysed in a French pressure cell at 1000 psi. The hexavalent protein was purified from the supernatant using Ni-NTA resin according to the protocol provided by the manufacturer (Qiagen). The elution buffer containing the protein was concentrated from 15 ml to 5 ml in a spin filter (Ultrafree-15, Millipore). Final purification was accomplished by gel filtration over Superdex 75 (prep grade, Pharmacia Biotech). The active fraction was identified by Western blots (Dale, J.B. and Beachey, E.H., "Multiple heart-cross-reactive epitopes of streptococcal M proteins," *J. Exp. Med. 161*:113-122, 1985) using rabbit antiserum against pep M24 (Beachey et al., "Purification and properties of M protein extracted from group A streptococci with pepsin: Covalent structure of the amino terminal region of the type 24 M antigen," *J. Exp. Med. 145*:1469-1483, 1977). Total protein concentration was determined by standard methods and the sample was diluted in PBS to contain 200 ug/ml of hexavalent protein. Purity of the samples was determined by gel scanning (Photoshop digital image and Collage image analysis).

### B. Analysis of the hexavalent vaccine

The structure of the hybrid emm gene was confirmed by doublestranded sequencing methods after ligation into pQE30. The sequence of each subunit was identical to the respective native emm gene (Figure 1). The fragments were joined only by the two amino acids specified by each unique restriction site used to facilitate their ligation (Figure 1).

The purified hexavalent protein migrated on SDS-polyacrylamide gels with an apparent M.W. of 45 kDa. (Figure 2). Gel scan analysis revealed that the intact hexavalent protein accounted for approximately 90% of the total stainable protein in the gel. Western blots using antisera against pep M24 showed that the majority of the remaining protein bands were immunoreactive and most likely were fragments of the hexavalent protein (data not shown).

### EXAMPLE 3

### IMMUNIZATION OF RABBITS, AND TESTING OF ANTISERA

### A. Immunization

Two groups of three rabbits each were immunized with 100 ug of hexavalent vaccine either precipitated with alum or emulsified in complete Freund's adjuvant. For precipitation in alum, the hexavalent protein (200 ug/ml) was added to an equal volume of aluminum hydroxide (2mg/ml) (Rehydragel HPA, Reheis, Inc., Berkeley Heights, NJ) and mixed gently at 40C overnight. The hexavalent protein was also emulsified in CFA at a final concentration of 100 ug/ml. Rabbits that received the hexavalent vaccine in alum were given 100 ug I.M. as an initial injection and the same dose was repeated at 4, and 8 weeks. The second set of rabbits received 100 ug of hexavalent vaccine in CFA subcutaneously as an initial injection and then booster injections of the same dose in saline were given at 4 and 8 weeks. Blood was obtained prior to the first injection and at 2-week intervals thereafter.

Antibody assays. ELISAs were performed using purified native pepsin-extracted M proteins (Beachey et al., "Purification and properties of M protein extracted from group A streptococci with pepsin: Covalent structure of the amino terminal region of the type 24 M antigen," *J. Exp. Med. 145*:1469-1483, 1977) or the purified hexavalent protein, as previously described (Dale et al., "Heterogeneity of type-specific and cross-reactive antigenic determinants within a single M protein of group A streptococci," *J. Exp. Med. 151*:1026-1038, 1980). Opsonic antibodies were detected by *in vitro* opsonization assays and indirect bactericidal assays (Beachey et al.. "Human immune response to immunization with a structurally defined polypeptide fragment of streptococcal M protein," *J. Exp. Med. 150*:862-877, 1979).

### B. Detection of M protein antibodies.

The preimmune and immune animal sera are assayed by ELISA using the vaccine protein and the native pepsin-extracted M proteins as solid-phase antigens (Dale et al., "Heterogeneity of type-specific and cross-reactive antigenic determinants within a single M protein of group A streptococci," *J. Exp. Med. 151*:1026-1038, 1980). ELISA titers are defined as the inverse of the last dilution of antisera resulting in an OD of >0.1 at 450nm. The titers of immune sera against the native M antigen are most likely to predict the levels of antibodies that are evoked by the recombinant protein that will react with the M protein on the surface of the respective serotype of streptococcus (i.e. promote opsonization).

### C. Detection of opsonic antibodies.

Opsonic M protein antibodies correlate with protection against infection with the same serotype of group A streptococci (Lancefield, R.C., "Current knowledge of the type specific M antigens of group A streptococci," *J. Immunol. 89*:307-313, 1962; Lancefield, R.C., "Persistence of type-specific antibodies in man following infection with group A streptococci," *J. Exp. Med. 110*:271-282, 1959). Two related in vitro assays are used to detect opsonic antibodies in immune sera. The first is a screening assay that measures opsonization in mixtures of immune serum, whole, nonimmune human blood and the test organism (Beachey et al., "Purification and properties of M protein extracted from group A streptococci with pepsin: Covalent structure of the amino terminal region of the type 24 M antigen," *J*. *Exp. Med. 145*:1469-1483, 1977). 0.1 ml of test serum is added to a standard number of bacteria and incubated for 15 minutes at room temperature. 0.4 ml of lightly heparinized human blood is added and the entire mixture is rotated end-over-end at 370 for 45 minutes. At the end of the rotation, smears are prepared on microscope slides that are air-dried and stained with Wright's stain. "Percent opsonization" is quantitated by counting the percentage of polymorphonuclear leukocytes that have ingested or are associated with bacteria. An interpretable assay must have a preimmune control value that is 10% opsonization or less.

Confirmation of the presence of opsonic antibodies is obtained by indirect bactericidal antibody assays according to the original description by Lancefield (Lancefield, R.C., "Current knowledge of the type specific M antigens of group A streptococci," *J. Immunol. 89*:307-313, 1962). This assay is performed using test mixtures as described above except that fewer bacteria are added and the rotation is allowed to proceed for 3 hours. At the end of the rotation, pour plates are made in sheep blood agar and bacteria surviving are quantitated after overnight growth at 370. Percent killing in the presence of immune serum is calculated by comparing to the growth in nonimmune serum.

### EXAMPLE 4

### MOUSE PROTECTION ASSAYS

### A. General Protocol

Protective efficacy of M protein vaccines is determined by either indirect or direct (passive or active immunization) mouse protection tests. Indirect tests are performed by giving mice 1 ml of immune or preimmune serum via the intraperitoneal (i.p.) route 24 hours prior to challenge infections with the test organism given i.p. (Beachey et al., "Human immune response to immunization with a structurally defined polypeptide fragment of streptococcal M protein," *J. Exp. Med. 150*:862-877, 1979). For each test organism, groups of 25 mice receive either preimmune of immune serum. The animals are then divided into 5 groups of 5 mice each and 10-fold increasing challenge doses of virulent streptococci are given to each subgroup. After 7 days of observation, the LD50 is calculated for each serotype tested.

Direct mouse protection tests are similarly performed except that mice are actively immunized with M protein vaccine prior to the challenge infections. Each mouse receives 25-50ug vaccine in alum given intramuscularly (i.m.) at time 0, 4 weeks, and 8 weeks. Challenge infections are performed ten weeks after the first injection. Control animals are sham immunized with alum alone. The LD50 is calculated and significance is determined using Fisher's exact test.

### B. Protection

In order to show directly the protective efficacy of opsonic antibodies evoked by the hexavalent vaccine, mice were immunized with the vaccine adsorbed to ALUM and then challenged with two of the serotypes represented in the vaccine. Female outbred white Swiss mice were immunized via the I.M. route in the hind leg according to the following schedule: time 0, 25ug; 3 weeks, 25 ug; 6 weeks, 50ug; and 13 weeks, 50ug. Challenge experiments were performed on the 20 immunized mice and 20 control, unimmunized mice (Table 1.) The challenge strains were types 24 and 19, with the reasoning that the M24 peptide is the largest fragment in the hexavalent protein and is reiterated and the M 19 fragment is one of two that are only 35 amino acids long. These two fragments should reflect the range of protective immunogenicity of the hexavalent protein. Interperitoneal challenge of mice with virulent streptococci is the most stringent laboratory assay for opsonic antibodies.

In this experiment, two groups of ten mice each were challenged with an inoculum that approximated the LD₇₀-LD₁₀₀ for each serotype, which was 2x10⁴ CFU. The challenge experiments were begun 15 weeks after the first dose of vaccine was administered and deaths were recorded for 10 days. The mice that were immunized with the hexavalent vaccine and challenged with type 24 streptococci were significantly protected from death compared to the control group (p=.0001). The mice challenged with type 19 streptococci were protected by vaccination, but the level was not statistically significant (p=15). Had the challenged group been twice the size, the same level of protection would have resulted in a statistically significant survival rate. When the survival of the entire immunized group of mice is analyzed, the level of protection was highly significant (p=.0002).

**Table 1. Protective immunogenicity of the hexavalent vaccine in mice that were challenged i.p. with virulent type 24 and type 19 streptococci**

| | #Dead/#Survived of Mice Challenged (% survival) | | |
|---|---|---|---|
| Group | Type 24 | Type 19 | Total |
| Immunized mice | 0/10 (100) | 4/6 (60) | 4/16 (80) p=.0002* |
| Control mice | 9/1 (10) | 7/3(30) | 16/4 (20) |

| | | | |
|---|---|---|---|
| *p value was calculated using the Fisher exact test. | | | |

### EXAMPLE 5

### ASSAYS FOR TISSUE-CROSSREACTIVE ANTIBODIES

To assure that none of the M protein vaccines evokes tissue-crossreactive antibodies, indirect immunofluorescence assays are performed using frozen sections of human heart, kidney, and brain (Dale, J.B. and Beachey E.H., "Protective antigenic determinant of streptococcal M protein shared with sarcolemmal membrane protein of human heart," *J. Exp. Med. 156*:1165-1176, 1982). Thin sections of tissue obtained at autopsy (4um) are prepared on microscope slides and stored in a sealed box at -700C until use. Test serum is diluted 1:5 in PBS and dropped onto the tissue section. Control slides are made with preimmune serum and PBS. The slides are incubated at ambient temperature for 30 minutes and then washed three times in PBS in a slide holder. Fluorescein-labeled goat anti-IgG/IgM/IgA is diluted 1:40 in PBS and dropped onto the slides which are again washed, dried, and mounted with 1% Gelvetol and a coverslip. Fluorescence is detected using a Zeiss Axiophot microscope equipped with a xenon light source. Immunofluorescence is recorded using a scale of 0-4+, with 0 being no fluorescence and 4+ being that obtained with a standard, positive antiserum raised in rabbits against whole type 5 M protein (Dale, J.B. and Beachey, E.H., "Multiple heart-cross-reactive epitopes of streptococcal M proteins," *J. Exp. Med. 161*:113-122, 1985).

### EXAMPLE 6

### COMPARISON OF THE IMMUNOGENICITY OF A HEXAVALENT VACCINE DELIVERED IN ALUM VERSUS FREUND'S ADJUVANT

Three rabbits each were immunized with 100 ug doses of the hexavalent vaccine in either alum of CFA. Booster injections of the same dose were given at 4 and 8 weeks in either alum or saline, respectively. ELISA titers were determined using the purified hexavalent protein as the solid phase antigen (Figure 3). Sera from the animals that received the hexavalent vaccine in alum had antibody titers that were equal to or greater than the sera from rabbits that received the same dose in CFA. In a subsequent experiment, three rabbits were immunized I.M. with 100 ug of the hexavalent vaccine in saline alone according the same schedule. None of these rabbits developed significant antibody titers against either the immunogen or the respective pep M proteins (data not shown). These data indicate that alum is a suitable and necessary adjuvant for the multivalent vaccine and is equal to the adjuvant activity of CFA in combination with the hexavalent protein.

### EXAMPLE 7

### PROTECTIVE IMMUNOGENICITY OF THE COMPONENT SUBUNITS OF A HEXAVALENT VACCINE

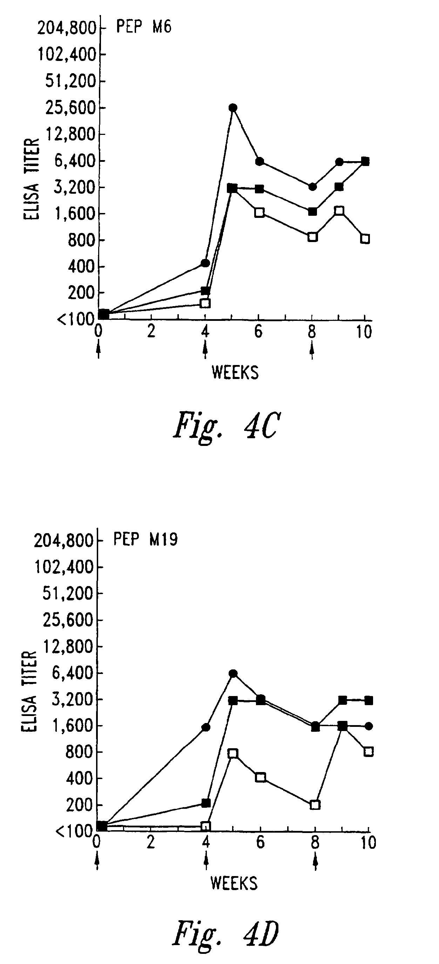

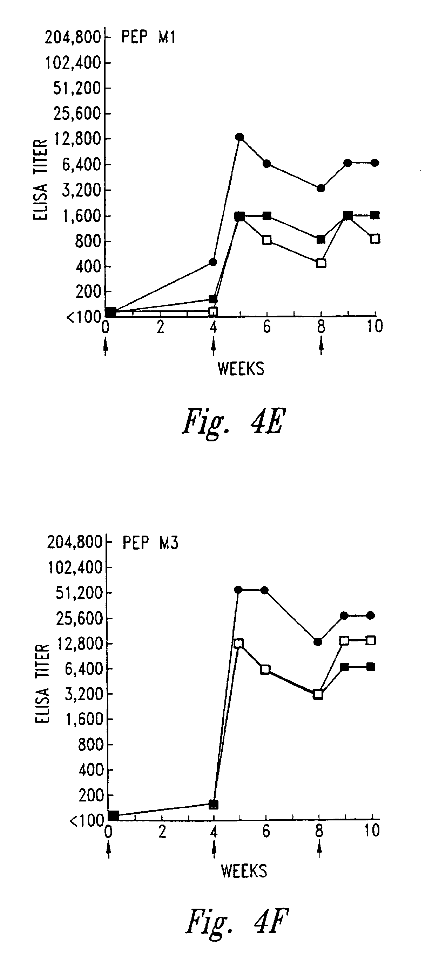

One of the major goals of this study was to design a multivalent, hybrid protein that retained the immunogenic properties of each M protein subunit. ELISAs were performed on sera obtained from the three rabbits immunized with the hexavalent vaccine in alum (Figure 4). In each case the ELISA antigen was the purified pepsin-extracted M protein. Thus, the assay measures only the antibodies evoked by the hexavalent protein that react with the native M protein and not the antibodies that may be specific for the joining segments or conformations that are not present in the native M proteins. The hexavalent protein evoked significant levels of antibodies against each M protein represented in the vaccine construct (Figure 4). Importantly, none of the antisera contained antibodies that crossreacted with human heart tissue or kidney tissue, as determined by indirect immunofluorescence assays (data not shown).

Sera from all three rabbits contained significant levels of opsonic antibodies against each serotype of group A streptococci represented in the vaccine (Figure 5). These results were confirmed by indirect bactericidal assays using one of the immune sera (Table 2). Taken together, the results indicate that the individual components of the hexavalent vaccine retain the conformation and immunogenicity necessary to elicit antibodies that react with the native M proteins on the surface of each respective serotype of group A streptococci.

**Table 2. Indirect bactericidal assay of rabbit antiserum against the hexavalent M protein vaccine.**

| | | CFU Surviving 3 hr rotation: | | |
|---|---|---|---|---|
| Serotype | Inoculum(CFU) | Preimmune | Immune | Percent Reduction |
| 24 | 12 | 2890 | 0 | 100 |
| 5 | 11 | 3260 | 0 | 100 |
| 6 | 6 | 2640 | 0 | 100 |
| 19 | 6 | 1580 | 0 | 100 |
| 1 | 8 | 2670 | 490 | 82 |
| 3 | 11 | 1720 | 10 | 99 |

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. A fusion polypeptide, comprising: (a) a multivalent immunogenic portion wherein the immunogenic portion comprises at least two immunogenic peptides, wherein each immunogenic peptide comprises at least 10 amino acids, of an amino-terminal fragment of a group A streptococcal M protein, and wherein the multivalent immunogenic portion is capable of eliciting an immune response against group A streptococci; and (b) a carboxy-terminal peptide that is a reiteration of a peptide from the amino-terminal region of the multivalent portion.

2. The fusion polypeptide according to claim 1 wherein only one immunogenic peptide is reiterated.

3. The fusion polypeptide according to claim 1 wherein the immune response against group A streptococci comprises opsonic antibodies that do not cross-react with human tissue.

4. The fusion polypeptide according to any one of claims 1-3 wherein the fusion polypeptide has at least one immunogenic peptide from a group A streptococci serotype selected from 1, 2, 3, 4, 5, 6, 11, 12, 13, 14, 18, 19, 22, 24, 28, 30, 48, 49, 52, 55, and 56.

5. The fusion polypeptide according to any one of claims 1-3 wherein at least one of the immunogenic peptides is from a serotype 1 group A streptococci.

6. The fusion polypeptide according to any one of claims 1-3 wherein at least one of the immunogenic peptides is from a serotype 2 group A streptococci.

7. The fusion polypeptide according to any one of claims 1-3 wherein at least one of the immunogenic peptides is from a serotype 3 group A streptococci.

8. The fusion polypeptide according to any one of claims 1-3 wherein at least one of the immunogenic peptides is from a serotype 5 group A streptococci.

9. The fusion polypeptide according to any one of claims 1-3 wherein at least one of the immunogenic peptides is from a serotype 6 group A streptococci.

10. The fusion polypeptide according to any one of claims 1-3 wherein at least one of the immunogenic peptides is from a serotype 11 group A streptococci.

11. The fusion polypeptide according to any one of claims 1-3 wherein at least one of the immunogenic peptides is from a serotype 12 group A streptococci.

12. The fusion polypeptide according to any one of claims 1-3 wherein at least one of the immunogenic peptides is from a serotype 13 group A streptococci.

13. The fusion polypeptide according to any one of claims 1-3 wherein at least one of the immunogenic peptides is from a serotype 14 group A streptococci.

14. The fusion polypeptide according to any one of claims 1-3 wherein at least one of the immunogenic peptides is from a serotype 18 group A streptococci.

15. The fusion polypeptide according to any one of claims 1-3 wherein at least one of the immunogenic peptides is from a serotype 19 group A streptococci.

16. The fusion polypeptide according to any one of claims 1-3 wherein at least one of the immunogenic peptides is from a serotype 22 group A streptococci.

17. The fusion polypeptide according to any one of claims 1-3 wherein at least one of the immunogenic peptides is from a serotype 24 group A streptococci.

18. The fusion polypeptide according to any one of claims 1-3 wherein at least one of the immunogenic peptides is from a serotype 28 group A streptococci.

19. The fusion polypeptide according to any one of claims 1-3 wherein the immunogenic portion consists of six to ten immunogenic peptides.

20. The fusion polypeptide according to any one of claims 1-3 wherein the immunogenic portion consists of six immunogenic peptides.

21. The fusion polypeptide according to any one of claims 1-3 and 19-20 wherein each immunogenic peptide of the immunogenic portion is from a different group A streptococcal serotype.

22. The fusion polypeptide according to claim 21 wherein the serotypes are 1, 3, 5, 6, 19, and 24.

23. The fusion polypeptide according to claim 21 wherein the serotypes are 1, 3, 5, 6, 18, 19, 22, 24, 28, and 30.

24. The fusion polypeptide according to any one of claims 1-23 wherein the immunogenic polypeptides of the fusion polypeptide are joined by amino acids encoded by a restriction enzyme site.

25. The fusion polypeptide according to any one of claims 1-24 further comprising a marker.

26. The fusion polypeptide according to claim 25 wherein the marker is capable of binding to nickel resin.

27. A composition, comprising a fusion polypeptide according to any one of claims 1-26 and a pharmaceutically acceptable excipient, carrier, stabilizer or diluent.

28. The composition according to claim 27, further comprising an adjuvant.

29. The composition according to claim 28 wherein the adjuvant is alum or Freund's adjuvant.

30. The composition according to any one of claims 27-29, further comprising an immunomodulatory cofactor.

31. The composition according to claim 30 wherein the immunomodulatory cofactor is selected from the group consisting of IL-4, IL-10, Y-IFN, IL-2, IL-12, and IL-15.

32. A fusion polypeptide according to any one of claims 1-26, or a composition according to any one of claims 27-31, for therapeutic use.

33. Use of a fusion polypeptide according to any one of claims 1-26, or a composition according to any one of claims 27-31, for the manufacture of a medicament for treating group A streptococci infections.

34. Use according to claim 33 wherein said fusion polypeptide or composition elicits opsonic antibodies.

35. Use according to claim 34 wherein said opsonic antibodies do not cross-react with host tissue.

## Patentansprüche

1. Fusionspolypeptid, umfassend:
(a) einen multivalenten immunogenen Anteil, wobei der immunogene Anteil mindestens zwei immunogene Peptide umfasst, wobei jedes immunogene Peptid mindestens 10 Aminosäuren eines aminoterminalen Fragments eines M-Proteins aus Gruppe A-Streptokokken umfasst, und wobei der multivalente immunogene Anteil fähig ist, eine lmmunreaktion gegen Gruppe A-Streptokokken der auszulösen; und
(b) ein carboxyterminales Peptid, bei dem es sich um die Wiederholung eines Peptids aus dem aminoterminalen Bereich des multivalenten Anteils handelt.

2. Fusionspolypeptid gemäß Anspruch 1, wobei nur ein immunogenes Peptid wiederholt wird.

3. Fusionspolypeptid gemäß Anspruch 1, wobei die lmmunreaktion gegen Gruppe A-Streptokokken opsonische Antikörper umfasst, die nicht mit menschlichem Gewebe kreuzreagieren.

4. Fusionspolypeptid gemäß einem der Ansprüche 1 - 3, wobei das Fusionspolypeptid mindestens ein immunogenes Peptid aus einem aus 1, 2, 3, 4, 5, 6, 11, 12, 13, 14, 18, 19, 22, 24, 28, 30, 48, 49, 52, 55 und 56 ausgewählten Serotyp von Gruppe A-Streptokokken aufweist.

5. Fusionspolypeptid gemäß einem der Ansprüche 1 - 3, wobei mindestens eines der immunogenen Peptide aus einer Gruppe A-Streptokokke des Serotyps 1 stammt.

6. Fusionspolypeptid gemäß einem der Ansprüche 1 - 3, wobei mindestens eines der immunogenen Peptide aus einer Gruppe A-Streptokokke des Serotyps 2 stammt.

7. Fusionspolypeptid gemäß einem der Ansprüche 1 - 3, wobei mindestens eines der immunogenen Peptide aus einer Gruppe A-Streptokokke des Serotyps 3 stammt.

8. Fusionspolypeptid gemäß einem der Ansprüche 1 - 3, wobei mindestens eines der immunogenen Peptide aus einer Gruppe A-Streptokokke des Serotyps 5 stammt.

9. Fusionspolypeptid gemäß einem der Ansprüche 1 - 3, wobei mindestens eines der immunogenen Peptide aus einer Gruppe A-Streptokokke des Serotyps 6 stammt.

10. Fusionspolypeptid gemäß einem der Ansprüche 1 - 3, wobei mindestens eines der immunogenen Peptide aus einer Gruppe A-Streptokokke des Serotyps 11 stammt.

11. Fusionspolypeptid gemäß einem der Ansprüche 1 - 3, wobei mindestens eines der immunogenen Peptide aus einer Gruppe A-Streptokokke des Serotyps 12 stammt.

12. Fusionspolypeptid gemäß einem der Ansprüche 1 - 3, wobei mindestens eines der immunogenen Peptide aus einer Gruppe A-Streptokokke des Serotyps 13 stammt.

13. Fusionspolypeptid gemäß einem der Ansprüche 1 - 3, wobei mindestens eines der immunogenen Peptide aus einer Gruppe A-Streptokokke des Serotyps 14 stammt.

14. Fusionspolypeptid gemäß einem der Ansprüche 1 - 3, wobei mindestens eines der immunogenen Peptide aus einer Gruppe A-Streptokokke des Serotyps 18 stammt.

15. Fusionspolypeptid gemäß einem der Ansprüche 1 - 3, wobei mindestens eines der immunogenen Peptide aus einer Gruppe A-Streptokokke des Serotyps 19 stammt.

16. Fusionspolypeptid gemäß einem der Ansprüche 1 - 3, wobei mindestens eines der immunogenen Peptide aus einer Gruppe A-Streptokokke des Serotyps 22 stammt.

17. Fusionspolypeptid gemäß einem der Ansprüche 1 - 3, wobei mindestens eines der immunogenen Peptide aus einer Gruppe A-Streptokokke des Serotyps 24 stammt.

18. Fusionspolypeptid gemäß einem der Ansprüche 1 - 3, wobei mindestens eines der immunogenen Peptide aus einer Gruppe A-Streptokokke des Serotyps 28 stammt.

19. Fusionspolypeptid gemäß einem der Ansprüche 1 - 3, wobei der immunogene Anteil aus sechs bis zehn immunogenen Peptiden besteht.

20. Fusionspolypeptid gemäß einem der Ansprüche 1 - 3, wobei der immunogene Anteil aus sechs immunogenen Peptiden besteht.

21. Fusionspolypeptid gemäß einem der Ansprüche 1 - 3 und 19 - 20, wobei jedes immunogene Peptid des immunogenen Anteils aus einem anderen Serotyp von Gruppe A-Streptokokken stammt.

22. Fusionspolypeptid gemäß Anspruch 21, wobei es sich bei den Serotypen um 1, 3, 5, 6, 19 und 24 handelt.

23. Fusionspolypeptid gemäß Anspruch 21, wobei es sich bei den Serotypen um 1, 3, 5, 6, 18, 19, 22, 24, 28 und 30 handelt.

24. Fusionspolypeptid gemäß einem der Ansprüche 1 - 23, wobei die immunogenen Polypeptide des Fusionspolypeptids durch Aminosäuren, die durch eine Restriktionsenzymstelle kodiert werden, verbunden sind.

25. Fusionspolypeptid gemäß einem der Ansprüche 1 - 24, ferner umfassend einen Marker.

26. Fusionspolypeptid gemäß Anspruch 25, wobei der Marker fähig ist, an Nickelharz zu binden.

27. Zusammensetzung, umfassend ein Fusionspolypeptid gemäß einem der Ansprüche 1 - 26 und einen pharmazeutisch verträglichen Exzipienten, Träger, Stabilisator oder ein pharmazeutisch verträgliches Verdünnungsmittel.

28. Zusammensetzung gemäß Anspruch 27, ferner umfassend ein Adjuvans.

29. Zusammensetzung gemäß Anspruch 28, wobei es sich bei dem Adjuvans um Alaun oder Freundsches Adjuvans handelt.

30. Zusammensetzung gemäß einem der Ansprüche 27 - 29, ferner umfassend einen immunmodulatorischen Kofaktor.

31. Zusammensetzung gemäß Anspruch 30, wobei der immunmodulatorische Kofaktor aus der Gruppe bestehend aus IL-4, IL-10, Y-IFN, IL-2, IL-12 und IL-15 ausgewählt ist.

32. Fusionspolypeptid gemäß einem der Ansprüche 1 - 26 oder Zusammensetzung gemäß einem der Ansprüche 27 - 31 zur therapeutischen Verwendung.

33. Verwendung eines Fusionspolypeptids gemäß einem der Ansprüche 1 - 26 oder einer Zusammensetzung gemäß einem der Ansprüche 27 - 31 zur Herstellung eines Medikaments zum Behandeln von Infektionen mit Gruppe A-Streptokokken.

34. Verwendung gemäß Anspruch 33 wobei das Fusionspolypeptid oder die Zusammensetzung opsonische Antikörper auslöst.

35. Verwendung gemäß Anspruch 34, wobei die opsonischen Antikörper nicht mit Wirtsgewebe kreuzreagieren.

## Revendications

1. Polypeptide de fusion qui comprend
(a) une partie immunogénique multivalente, la partie immunogénique comprend au moins deux peptides immunogéniques, chaque peptide immunogénique comprend au moins 10 acides aminés d'un fragment amino terminal d'une protéine M streptococcique de groupe A et la partie immunogénique multivalente peut provoquer une réponse immunitaire contre des streptocoques du groupe A et
(b) un peptide carboxy terminal qui répète un peptide situé dans la zone amino terminale de la partie multivalente.

2. Polypeptide de fusion selon la revendication 1, dans lequel un seul peptide immunogénique est répété.

3. Polypeptide de fusion selon la revendication 1, dans lequel la réponse immunitaire contre les streptocoques du groupe A comprend des anticorps opsoniques qui ne peuvent pas avoir de réaction croisée avec des tissus humains.

4. Polypeptide de fusion selon l'une des revendications 1 à 3, dans lequel le polypeptide de fusion a au moins un peptide immunogénique qui provient d'un sérotype des streptocoques du groupe A parmi les suivants : 1, 2, 3, 4, 5, 6, 11, 12, 13, 14, 18, 19, 22, 24, 28, 30, 48, 49, 52, 55 et 56.

5. Polypeptide de fusion selon l'une des revendications 1 à 3, dans lequel au moins un des peptides immunogéniques provient de streptocoques du groupe A de sérotype 1.

6. Polypeptide de fusion selon l'une des revendications 1 à 3, dans lequel au moins un des peptides immunogéniques provient de streptocoques du groupe A de sérotype 2.

7. Polypeptide de fusion selon l'une des revendications 1 à 3, dans lequel au moins un des peptides immunogéniques provient de streptocoques du groupe A de sérotype 3.

8. Polypeptide de fusion selon l'une des revendications 1 à 3, dans lequel au moins un des peptides immunogéniques provient de streptocoques du groupe A de sérotype 5.

9. Polypeptide de fusion selon l'une des revendications 1 à 3, dans lequel au moins un des peptides immunogéniques provient de streptocoques du groupe A de sérotype 6.

10. Polypeptide de fusion selon l'une des revendications 1 à 3, dans lequel au moins un des peptides immunogéniques provient de streptocoques du groupe A de sérotype 11.

11. Polypeptide de fusion selon l'une des revendications 1 à 3, dans lequel au moins un des peptides immunogéniques provient de streptocoques du groupe A de sérotype 12.

12. Polypeptide de fusion selon l'une des revendications 1 à 3, dans lequel au moins un des peptides immunogéniques provient de streptocoques du groupe A de sérotype 13.

13. Polypeptide de fusion selon l'une des revendications 1 à 3, dans lequel au moins un des peptides immunogéniques provient de streptocoques du groupe A de sérotype 14.

14. Polypeptide de fusion selon l'une des revendications 1 à 3, dans lequel au moins un des peptides immunogéniques provient de streptocoques du groupe A de sérotype 18.

15. Polypeptide de fusion selon l'une des revendications 1 à 3, dans lequel au moins un des peptides immunogéniques provient de streptocoques du groupe A de sérotype 19.

16. Polypeptide de fusion selon l'une des revendications 1 à 3, dans lequel au moins un des peptides immunogéniques provient de streptocoques du groupe A de sérotype 22.

17. Polypeptide de fusion selon l'une des revendications 1 à 3, dans lequel au moins un des peptides immunogéniques provient de streptocoques du groupe A de sérotype 24.

18. Polypeptide de fusion selon l'une des revendications 1 à 3, dans lequel au moins un des peptides immunogéniques provient de streptocoques du groupe A de sérotype 28.

19. Polypeptide de fusion selon l'une des revendications 1 à 3, dans lequel la partie immunogénique comporte de six à dix peptides immunogéniques.

20. Polypeptide de fusion selon l'une des revendications 1 à 3, dans lequel la partie immunogénique comporte six peptides immunogéniques.

21. Polypeptide de fusion selon l'une des revendications 1 à 3 et 19 à 20, dans lequel chaque peptide immunogénique de la partie immunogénique provient d'un sérotype streptococcique différent de groupe A.

22. Polypeptide de fusion selon la revendication 21, dans lequel les sérotypes sont 1, 3, 5, 6, 19 et 24.

23. Polypeptide de fusion selon la revendication 21, dans lequel les sérotypes sont 1, 3, 5, 6, 18, 19, 22, 24, 28 et 30.

24. Polypeptide de fusion selon l'une des revendications 1 à 23, dans lequel les polypeptides immunogéniques du polypeptide de fusion sont fixés par des acides aminés codés par un site d'enzyme de restriction.

25. Polypeptide de fusion selon l'une des revendications 1 à 24, comportant en outre un marqueur.

26. Polypeptide de fusion selon la revendication 25, dans lequel le marqueur peut se lier à la résine de nickel.

27. Composé qui comprend un polypeptide de fusion selon l'une des revendications 1 à 26 et un excipient, un porteur, un stabilisateur ou un diluant acceptables pour un usage pharmaceutique.

28. Composé selon la revendication 27, comportant en outre un adjuvant.

29. Composé selon la revendication 28, dans lequel l'adjuvant est l'alun ou l'adjuvant de Freund.

30. Composé selon l'une des revendications 27 à 29, comportant en outre un cofacteur immunomodulateur.

31. Composé selon la revendication 30, dans lequel le cofacteur immunomodulateur est choisi parmi le groupe suivant : l'interleukine-4, l'interleukine-10, l'interféron gamma, l'interleukine-2, l'interleukine-12 et l'interleukine-15.

32. Polypeptide de fusion selon l'une des revendications 1 à 26 ou un composé selon l'une des revendications 27 à 31 pour une utilisation thérapeutique.

33. Utilisation d'un polypeptide de fusion selon l'une des revendications 1 à 26 ou d'un composé selon l'une des revendications 27 à 31 pour fabriquer un médicament destiné à traiter des infections dues à des streptocoques du groupe A.

34. Utilisation selon la revendication 33, d'après laquelle ledit polypeptide de fusion ou ledit composé produit des anticorps opsoniques.

35. Utilisation selon la revendication 34, d'après laquelle lesdits anticorps opsoniques n'ont pas de réaction croisée avec les tissus hôte.


### SEQUENCE LISTING

(1) GENERAL INFORMATION:
   (i) APPLICANT: Dale. James B.
   (ii) TITLE OF INVENTION: GROUP A STREPTOCOCCAL VACCINES
   (iii) NUMBER OF SEQUENCES: 16
   (iv) CORRESPONDENCE ADDRESS:
      (A) ADDRESSEE: SEED and BERRY LLP
      (B) STREET: 6300 Columbia Center, 701 Fifth Avenue
      (C) CITY: Seattle
      (D) STATE: Washington
      (E) COUNTRY: USA
      (F) ZIP: 98104
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0. Version #1.30
   (vi) CURRENT APPLICATION DATA:
      (A) APPLICATION NUMBER: US
      (B) FILING DATE: 12-SEP-1997
      (C) CLASSIFICATION:
   (viii) ATTORNEY/AGENT INFORMATION:
      (A) NAME: McMasters. David D.
      (B) REGISTRATION NUMBER: 33.963
      (C) REFERENCE/DOCKET NUMBER: 481112.410P1
   (ix) TELECOMMUNICATION INFORMATION:
      (A) TELEPHONE: (206) 622-4900
      (B) TELEFAX: (206) 682-6031
(2) INFORMATION FOR SEQ ID NO:1:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:1:
      GGGGGGGCAT CGGTCGCGAC TAGGTCTCAG ACAGAT 36
(2) INFORMATION FOR SEQ ID NO:2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:2:
      GGGGGGGGAT CCACGTAGTT TCTCTTTAGC 30
(2) INFORMATION FOR SEQ ID NO:3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:3:
      GGGGGGGGAT CCGCCGTGAC TAGGGGTACA 30
(2) INFORMATION FOR SEQ ID NO:4:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:4:
      GGGGGGGTCG ACCTCAGTTT TTAACCCTTC 30
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:5:
      GGGGGGGTCG ACAGAGTGTT TCCTAGGGGG 30
(2) INFORMATION FOR SEQ ID NO:6:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:6:
      GGGGGGCCAT GGTAACTTGT CATTATTAGC 30
(2) INFORMATION FOR SEQ ID NO:7:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:7:
      GGGGGGCCAT GGAGAGTGCG TTATACTAGG 30
(2) INFORMATION FOR SEQ ID NO:8:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:8:
      GGGGGGCTGC AGAGATAACT TCTCATTCTG 30
(2) INFORMATION FOR SEQ ID NO:9:
   (i) SEQUENCE CHARACTERISTICS:
      (A) LENGTH: 30 base pairs
      (B) TYPE: nucleic acid
      (C) STRANDEONESS: single
      (D) TOPOLOGY: linear
   (xi) SEQUENCE DESCRIPTION: SEQ ID NO:9:
      GGGGGGCTGC AGAACGGTGA TGGTAATCCT 30
(2) INFORMATION FOR SEQ ID NO:10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:10:
      GGGGGGGGTA CCAGCTCTCT TAAAATCTCT 30
(2) INFORMATION FOR SEQ ID NO:11:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:11:
      GGGGGGGGTA CCTTGTTAGA TCAGGTTACA 30
(2) INFORMATION FOR SEQ ID NO:12:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:12:
      GGGGGGATCG ATATTTAACT CTTGTAACAG 30
(2) INFORMATION FOR SEQ ID NO:13:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:13:
      GGGGGGATCG ATGTCGCGAC TAGGTCTCAG 30
(2) INFORMATION FOR SEQ ID NO:14:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:14:
      GGGGGGAAGC TTTTACTTAC GTGCCTCTAA TTC 33
(2) INFORMATION FOR SEQ ID NO:15:
   (i) SEQUENCE CHARACTERISTICS:
      (A) LENGTH: 1158 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..1149
   (xi) SEQUENCE DESCRIPTION: SEQ ID NO:15:
   GCA TGC ATG GTC GCG ACT AGG TCT CAG ACA GAT ACT CTG GAA AAA GTA      48
   Ala Cys Met Val Ala Thr Arg Ser Gln Thr Asp Thr Leu Glu Lys Val
     1               5                  10                  15
   CAA GAA CGT GCT GAC AAG TTT GAG ATA GAA AAC AAT ACG TTA AAA CTT      96
   Gln Glu Arg Ala Asp Lys Phe Glu Ile Glu Asn Asn Thr Leu Lys Leu
                20                  25                  30
   AAG AAT AGT GAC TTA AGT TTT AAT AAT AAA GCG TTA AAA GAT CAT AAT     144
   Lys Asn Ser Asp Leu Ser Phe Asn Asn Lys Ala Leu Lys Asp His Asn
            35                  40                  45
   GAT GAG TTA ACT GAA GAG TTG AGT AAT GCT AAA GAG AAA CTA CGT GGA     192
   Asp Glu Leu Thr Glu Glu Leu Ser Asn Ala Lys Glu Lys Leu Arg Gly
        50                  55                  60
   TCC GCC GTG ACT AGG GGT ACA ATA AAT GAC CCG CAA AGA GCA AAA GAA     240
   Ser Ala Val Thr Arg Gly Thr Ile Asn Asp Pro Gln Arg Ala Lys Glu
    65                  70                  75                  80
   GCT CTT GAC AAG TAT GAG CTA GAA AAC CAT GAC TTA AAA ACT AAG AAT     288
   Ala Leu Asp Lys Tyr Glu Leu Glu Asn His Asp Leu Lys Thr Lys Asn
                    85                  90                  95
   GAA GGG TTA AAA ACT GAG AAT GAA GGG TTA AAA ACT GAG AAT GAA GGG     336
   Glu Gly Leu Lys Thr Glu Asn Glu Gly Leu Lys Thr Glu Asn Glu Gly
               100                 105                 110
   TTA AAA ACT GAG AAT GAA GGG TTA AAA ACT GAG GTC GAC AGA GTG TTT     384
   Leu Lys Thr Glu Asn Glu Gly Leu Lys Thr Glu Val Asp Arg Val Phe
           115                 120                 125
   CCT AGG GGG ACG GTA GAA AAC CCG GAC AAA GCA CGA GAA CTT CTT AAC     432
   Pro Arg Gly Thr Val Glu Asn Pro Asp Lys Ala Arg Glu Leu Leu Asn
   130 135 140
   AAG TAT GAC GTA GAG AAC TCT ATG TTA CAA GCT AAT AAT GAC AAG TTA 480
   Lys Tyr Asp Val Glu Asn Ser Met Leu Gln Ala Asn Asn Asp Lys Leu
   145 150 155 160
   CCA TGG AGA GTG CGT TAT ACT AGG CAT ACG CCA GAA GAT AAG CTA AAA 528
   Pro Trp Arg Val Arg Tyr Thr Arg His Thr Pro Glu Asp Lys Leu Lys
   165 170 175
   AAA ATT ATT GAC GAT CTT GAC GCA AAA GAA CAT GAA TTA CAA CAA CAG 576
   Lys Ile Ile Asp Asp Leu Asp Ala Lys Glu His Glu Leu Gln Gln Gln
   180 185 190
   AAT GAG AAG TTA TCT CTG CAG AAC GGT GAT GGT AAT CCT AGG GAA GTT 624
   Asn Glu Lys Leu Ser Leu Gln Asn Gly Asp Gly Asn Pro Arg Glu Val
   195 200 205
   ATA GAA GAT CTT GCA GCA AAC AAT CCC GCA ATA CAA AAT ATA CGT TTA 672
   Ile Glu Asp Leu Ala Ala Asn Asn Pro Ala Ile Gln Asn Ile Arg Leu
   210 215 220
   CGT CAC GAA AAC AAG GAC TTA AAA GCG AGA TTA GAG AAT GCA ATG GAA 720
   Arg His Glu Asn Lys Asp Leu Lys Ala Arg Leu Glu Asn Ala Met Glu
   225 230 235 240
   GTT GCA GGA AGA GAT TTT AAG AGA GCT GGT ACC TTG TTA GAT CAG GTT 768
   Val Ala Gly Arg Asp Phe Lys Arg Ala Gly Thr Leu Leu Asp Gln Val
   245 250 255
   ACA CAA TTA TAT ACT AAA CAT AAT AGT AAT TAC CAA CAA TAT AAT GCA 816
   Thr Gln Leu Tyr Thr Lys His Asn Ser Asn Tyr Gln Gln Tyr Asn Ala
   260 265 270
   CAA GCT GGC AGA CTT GAC CTG AGA CAA AAG GCT GAA TAT CTA AAA GGC 864
   Gln Ala Gly Arg Leu Asp Leu Arg Gln Lys Ala Glu Tyr Leu Lys Gly
   275 280 285
   CTT AAT GAT TGG GCT GAG AGG CTG TTA CAA GAG TTA AAT ATC GAT GTC 912
   Leu Asn Asp Trp Ala Glu Arg Leu Leu Gln Glu Leu Asn Ile Asp Val
   290 295 300
   GCG ACT AGG TCT CAG ACA GAT ACT CTG GAA AAA GTA CAA GAA CGT GCT 960
   Ala Thr Arg Ser Gln Thr Asp Thr Leu Glu Lys Val Gln Glu Arg Ala
   305 310 315 320
   GAC AAG TTT GAG ATA GAA AAC AAT ACG TTA AAA CTT AAG AAT AGT GAC      1008
   Asp Lys Phe Glu Ile Glu Asn Asn Thr Leu Lys Leu Lys Asn Ser Asp
                   325                 330                 335
   TTA AGT TTT AAT AAT AAA GCG TTA AAA GAT CAT AAT GAT GAG TTA ACT      1056
   Leu Ser Phe Asn Asn Lys Ala Leu Lys Asp His Asn Asp Glu Leu Thr
               340                 345                 350
   GAA GAG TTG AGT AAT GCT AAA GAG AAA CTA CGT AAA AAT GAT AAA TCA      1104
   Glu Glu Leu Ser Asn Ala Lys Glu Lys Leu Arg Lys Asn Asp Lys Ser
           355                 360                 365
   CTA TCT GAA AAA GCT AGT AAA ATT CAA GAA TTA GAG GCA CGT AAG          1149
   Leu Ser Glu Lys Ala Ser Lys Ile Gln Glu Leu Glu Ala Arg Lys
       370                 375                 380
   TAAAAGCTT                                                            1158
(2) INFORMATION FOR SEQ ID NO:16:
   (i) SEQUENCE CHARACTERISTICS:
      (A) LENGTH: 383 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Ala Cys Met Val Ala Thr Arg Ser Gln Thr Asp Thr Leu Glu Lys Val
    1               5                  10                  15
   Gln Glu Arg Ala Asp Lys Phe Glu Ile Glu Asn Asn Thr Leu Lys Leu
               20                  25                  30
   Lys Asn Ser Asp Leu Ser Phe Asn Asn Lys Ala Leu Lys Asp His Asn
           35                  40                  45
   Asp Glu Leu Thr Glu Glu Leu Ser Asn Ala Lys Glu Lys Leu Arg Gly
       50                  55                  60
   Ser Ala Val Thr Arg Gly Thr Ile Asn Asp Pro Gln Arg Ala Lys Glu
   65                  70                  75                  80
   Ala Leu Asp Lys Tyr Glu Leu Glu Asn His Asp Leu Lys Thr Lys Asn
   85 90 95
   Glu Gly Leu Lys Thr Glu Asn Glu Gly Leu Lys Thr Glu Asn Glu Gly
   100 105 110
   Leu Lys Thr Glu Asn Glu Gly Leu Lys Thr Glu Val Asp Arg Val Phe
   115 120 125
   Pro Arg Gly Thr Val Glu Asn Pro Asp Lys Ala Arg Glu Leu Leu Asn
   130 135 140
   Lys Tyr Asp Val Glu Asn Ser Met Leu Gln Ala Asn Asn Asp Lys Leu
   145 150 155 160
   Pro Trp Arg Val Arg Tyr Thr Arg His Thr Pro Glu Asp Lys Leu Lys
   165 170 175
   Lys Ile Ile Asp Asp Leu Asp Ala Lys Glu His Glu Leu Gln Gln Gln
   180 185 190
   Asn Glu Lys Leu Ser Leu Gln Asn Gly Asp Gly Asn Pro Arg Glu Val
   195 200 205
   Ile Glu Asp Leu Ala Ala Asn Asn Pro Ala Ile Gln Asn Ile Arg Leu
   210 215 220
   Arg His Glu Asn Lys Asp Leu Lys Ala Arg Leu Glu Asn Ala Met Glu
   225 230 235 240
   Val Ala Gly Arg Asp Phe Lys Arg Ala Gly Thr Leu Leu Asp Gln Val
   245 250 255
   Thr Gln Leu Tyr Thr Lys His Asn Ser Asn Tyr Gln Gln Tyr Asn Ala
   260 265 270
   Gln Ala Gly Arg Leu Asp Leu Arg Gln Lys Ala Glu Tyr Leu Lys Gly
   275 280 285
   Leu Asn Asp Trp Ala Glu Arg Leu Leu Gln Glu Leu Asn Ile Asp Val
   290 295 300
   Ala Thr Arg Ser Gln Thr Asp Thr Leu Glu Lys Val Gln Glu Arg Ala
   305 310 315 320
   Asp Lys Phe Glu Ile Glu Asn Asn Thr Leu Lys Leu Lys Asn Ser Asp
   325 330 335
   Leu Ser Phe Asn Asn Lys Ala Leu Lys Asp His Asn Asp Glu Leu Thr
   340 345 350
   Glu Glu Leu Ser Asn Ala Lys Glu Lys Leu Arg Lys Asn Asp Lys Ser
   355 360 365
   Leu Ser Glu Lys Ala Ser Lys Ile Gln Glu Leu Glu Ala Arg Lys
   370 375 380